# EUROPEAN PATENT APPLICATION

(11) **EP 3 311 861 A1**
(43) Date of publication of application: **25.04.2018**
(21) Application number: 17196583.3
(22) Date of filing: 16.10.2017
(51) Int. Cl.: A61M 1/16, B01D 63/10, C02F 1/44, C02F 9/00

(54) **DIALYSATE REGENERATING DEVICE**

(30) Priority: 18.10.2016 US 201662409544 P
(71) Applicant: Cook Medical Technologies LLC, Bloomington, IN 47404 (US)
(72) Inventor: DOWELL, Angela R., Lafayette, IN Indiana 47904 (US)
(74) Representative: Garratt, Peter Douglas

(57) **Abstract**

Devices and methods for regenerating dialysate are disclosed. In certain aspects, the present disclosure provides a multi-stage dialysate regenerating device comprising a first stage having a filter membrane for filtering a dialysate solution into a first fluid flow and a second fluid flow and a second stage having an osmotic membrane for allowing transfer of solvent from one of the first or second fluid flow into the other. Multi-stage dialysate regenerating devices with at least one stage operating by forward-osmosis are also disclosed.

## Description

### BACKGROUND

The present disclosure pertains generally to devices and methods for regenerating dialysate. In certain aspects, the present disclosure provides a multi-stage dialysate regenerating device comprising a first stage having a filter membrane for filtering a dialysate solution into a first fluid flow path and a second fluid flow path and a second stage having an osmotic membrane for allowing transfer of solvent (e.g., water) from one of the first or second fluid flow paths into the other. In certain embodiments, the present disclosure provides a multi-stage dialysate regenerating device with at least one stage operating by forward-osmosis.

Patients suffering from kidney failure or reduced kidney function are often treated by dialysis. Dialysis supplements or replaces kidney function, promoting a healthy equilibrium of water and minerals (sodium, potassium, chloride, calcium, phosphorus, magnesium, and sulfate) in the blood of a patient and removing waste and excess water from the blood.

Dialysis machines use diffusion and/or ultrafiltration to remove solutes and/or fluid from the blood. Diffusion and/or ultrafiltration occur by a fluid passing over and/or through one or more membranes. There are several types of dialysis, including hemodialysis, peritoneal dialysis, hemofiltration, hemodiafiltration, and intestinal dialysis. Some forms of dialysis circulate blood over and/or through a membrane positioned outside of the body of the patient (*e.g.,* hemodialysis). Other forms of dialysis circulate blood over and/or through a membrane of the patient, such as the peritoneal membrane (*i.e.,* peritoneal dialysis).

During dialysis, a semipermeable membrane separates the patient's blood from a dialysis fluid (*i.e.,* "dialysate"), allowing waste products such as urea and creatinine can pass from the blood through the membrane and into the dialysis fluid by means of diffusion. Other solutes, such as bicarbonate, may pass from the dialysate through the membrane and into the blood.

Present-day patients undergoing dialysis treatment may visit a hospital or dialysis center every few days to undergo a dialysis procedure that can last upwards of four hours and can use hundreds of liters of dialysate. Patients having in-home dialysis machines are able to undergo shorter treatments each day or even multiple times a day. In some instances, patients undergo peritoneal dialysis at home, such as overnight. Depending on the agents used in the dialysate, some patients undergoing peritoneal dialysis may experience a loss of filtration efficiency such as glycosylation of the peritoneal membrane due to glucose in the dialysate. Higher molecular weight dialysate can help reduce glycosylation but is more expensive than glucose as an osmotic agent in dialysate. Therefore, there remains a desire for improvement in this field.

### SUMMARY

The present disclosure pertains generally to devices and methods for regenerating dialysate. In certain aspects, the present disclosure provides a multi-stage dialysate regenerating device comprising a first stage having a filter membrane for filtering a dialysate solution into a first fluid flow and a second fluid flow and a second stage having an osmotic membrane for allowing transfer of solvent from one of the first or second fluid flow into the other.

In at least one embodiment of the present disclosure, a dialysate regenerating device includes a first stage having a first stage filter membrane having a pore size sufficient to separate large molecules, particularly the dialysate of interest, from the smaller molecules and material removed from the blood of the patient (e.g., urea, creatinine, and/or excess water). Such a filter membrane may have a nominal molecular weight limit (NMWL) or molecular weight cutoff (MWCO) of 500 kDa or less. In some instances, the filter membrane has a nominal molecular weight limit (NMWL) or molecular weight cutoff (MWCO) of 100 kDa or less. And, in some particular embodiments, the filter membrane has a nominal molecular weight limit (NMWL) or molecular weight cutoff (MWCO) of 10 kDa. Similarly, in some configurations, the filter membrane has an average pore size of 20 nanometers or less and/or an average pore size of 6 nanometers or more.

The second stage of the device includes an osmotic membrane. The osmotic membrane is arranged to allow the diffusion of solvent from one side of the membrane to the other. In several embodiments, the osmotic membrane allows for the transfer of water across the membrane while preventing the transfer of larger molecules. In arrangements of the present disclosure, the osmotic membrane allows the transfer of water from the filtrate of the first stage through the osmotic membrane into the retentate from the first stage while preventing substances such as urea and creatinine from crossing the membrane. The osmotic membrane has an average pore size of 20 nanometers or less, 5 nanometers or less, 1 nanometer or less, 5 Angstroms or less and/or 3 Angstroms or more.

In certain embodiments, the present disclosure provides a multi-stage dialysate regenerating device with at least one stage operating or facilitated by forward-osmosis. Forward-osmosis is the movement of solvent molecules through a semi-permeable membrane from a lower osmolarity (i.e., higher solvent concentration) into a region of higher osmolarity (i.e., lower solvent concentration), the semi-permeable membrane being permeable to the solvent and not the solute. Reverse-osmosis is the movement of solvent molecules through a semi-permeable membrane from a higher osmolarity (i.e., lower solvent concentration) into a region of lower osmolarity (i.e., higher solvent concentration). Forward-osmosis can occur without the application of external force. Reverse-osmosis, however, requires an external force (i.e., pressure) in excess of the osmotic pressure to force the solvent through the semi-permeable membrane from an area of higher osmolarity to lower osmolarity.

In certain aspects, the present disclosure provides a device for regenerating a dialysate fluid, comprising a device body defining a dialysate fluid inlet, a first stage, a second stage, a first outlet, and a second outlet. The first stage has a filter membrane separating a first fluid flow path from a second fluid flow path, the first fluid flow path extending from the first stage to the first outlet and the second fluid flow path extending from the first stage to the second outlet. The second stage has an osmotic membrane separating the first fluid flow path from the second fluid flow path. Wherein dialysate fluid enters the dialysate fluid inlet, passes through the first stage which separates the dialysate fluid into the first fluid flow path and the second fluid flow path, and the flow paths pass through the second stage which allows solvent to pass through the osmotic membrane, such as from the second fluid flow path into the first fluid flow path or from the body of the patient into the second fluid flow path.

In some arrangements, the first fluid flow path extends in a direction opposing that of the second fluid flow path. Additionally or alternatively, the second fluid flow path extends helically around the first fluid flow path or the first fluid flow path extends helically around the second fluid flow path. In some instances, the first and second fluid flow paths each extend helically so as to form a double helix.

In some instances, the portion of the dialysate fluid passing through the filter membrane enters the second fluid flow path and a portion of dialysate fluid that does not pass through the filter membrane continues into the first fluid flow path. Alternatively, the portion of the dialysate fluid passing through the filter membrane enters the first fluid flow path and a portion of dialysate fluid that does not pass through the filter membrane continues into the second fluid flow path.

Other arrangements of the present disclosure are contemplated. For instance, the first stage and/or second stage of the device may include a series of stacked or layered membranes arranged parallel to and/or transverse to the direction of fluid flow, allowing fluid to flow along, around, over, across, and/or between the membranes. In some arrangements, one or more membranes of the first and/or second stage may be arranged to define a fluid channel for receiving filtrate or permeate fluid. For example, the first and/or second stage may have one or more membranes arranged similar to book lungs found in arachnids.

The devices of the present disclosure may be stationary, portable, or implantable. In some instances, the device may be arranged for implantation within the abdominal cavity of a patient. In particular, the device may be arranged for implantation within the peritoneal cavity.

In some arrangements, the device body has an outer surface allowing water transport from the first fluid flow path or second fluid flow path to an environment adjacent the device body or vice versa. For instance, a portion of the second stage (e.g., the osmotic membrane) can be arranged for exposure to the peritoneal environment upon implantation within the body of a patient. In other arrangements, the second stage of the device is encapsulated within an impermeable membrane to prevent water transport, if desired, between the body of the patient and retentate fluid or permeate fluid of the dialysate. An encapsulated device may be desirable, in particular, for portable, non-implantable devices.

The disclosed devices and methods have been contemplated for use with a dialysate having a polyglycol, which includes water soluble polymers having repeating units represented by -(CH2CH2O)-. For example, polyglycols include polyethylene glycol and branched polymers. Polyglycols include terminal hydroxyl, aldehydes, carboxylic acid groups and/or other functional groups that are capable of forming association with blood borne waste products (e. g., urea). National formulary (NF) grades of these materials having a molecular weight of about 3000 to about 4000 are preferred but can be up to 20000. Suitable branched polymers include multiple branches of repeating units represented by - (CH2CH20)- and can be produced by polymerizing, grafting or otherwise reacting individual ethylene oxide groups, or polymers or pre-polymers thereof with polyhydric alcohols (e. g., glycerol, carbohydrates and the like), polyhydroxy aldehydes, or polyhydroxy ketones. Polyglycols can also be formed through use of polymers based on the monomer epichlorohydrin wherein the organic chloride groups of the resulting polymer are derivitized with amines to form quaternary amine groups.

An exemplary dialysate useful in the disclosed devices is an icodextrin electrolyte solution, such as the product sold under the name EXTRANEAL (icodextrin) Peritoneal Dialysis Solution by Baxter as of the filing date of this application. EXTRANEAL includes 7.5% Icodextrin in an electrolyte solution with 40 mEq/L lactate.

In some instances, the devices are included with and/or incorporate a pump for forcing dialysate fluid through the device. For example, the device may incorporate or communicate with an implantable pump having a magnetic or induction powered impeller. In some instances, the device may rely upon gravity or osmotic pressure to move dialysate fluid through the device.

The device and/or pump can be implanted and/or portable outside the body of the patient. In instances where the device and/or pump are portable and outside the body of the patient, the device and/or pump may be wearable by the patient in a small pack, with the device connected to a patient, such as to the patient's peritoneum via an implanted peritoneal dialysis catheter. In some instances, the device is part of an automatic peritoneal dialysis cycler system for cleaning and regenerating dialysate by separating the dialysate from smaller molecules which become the waste stream. Also, the device maybe part of a cartridge used in-line with gravity drain-and-fill methods of peritoneal dialysis dialysate exchange.

Methods of regenerating dialysate are also provided in the present disclosure. One exemplary method includes filtering a dialysate into a permeate fluid and retentate fluid with a filter membrane having a nominal molecular weight limit (NMWL) or molecular weight cutoff (MWCO) of 500 kDa or less; and passing water from the permeate fluid into the retentate fluid through an osmotic membrane separate from the filter membrane and having a smaller average pore size. In some instances, passing water from the permeate fluid into the retentate fluid through an osmotic membrane includes passing water from the permeate fluid into the retentate fluid using forward osmosis or partial forward osmosis combined with other means such as fluid pressure. The elements disclosed above are also contemplated for inclusion in the methods. For example, the filter membrane may have a nominal molecular weight limit (NMWL) or molecular weight cutoff (MWCO) of 100 kDa or less or of 10 kDa or less. Similarly, the filter membrane may have an average pore size of 20 nanometers or less and/or 6 nanometers or more. The osmotic membrane may have an average pore size of 20 nanometers or less, 5 nanometers or less, 1 nanometer or less, 5 Angstroms or less and/or 3 Angstroms or more.

In some instances, the methods include the passing of water from the patient into the permeate fluid. This may be desired for a patient predisposed to water retention, hypertension, and edema.

With respect to the specification and claims, it should be noted that the singular forms "a", "an", "the", and the like include plural referents unless expressly discussed otherwise. As an illustration, references to "a device" or "the device" include one or more of such devices and equivalents thereof. It also should be noted that directional terms, such as "up", "down", "top", "bottom", and the like, are used herein solely for the convenience of the reader in order to aid in the reader's understanding of the illustrated embodiments, and it is not the intent that the use of these directional terms in any manner limit the described, illustrated, and/or claimed features to a specific direction and/or orientation.

The language used in the claims and the written description and in the above definitions is to only have its plain and ordinary meaning, except for terms explicitly defined above. Such plain and ordinary meaning is defined here as inclusive of all consistent dictionary definitions from the most recently published (on the filing date of this document) general purpose Merriam-Webster dictionary.

As used in the claims and the specification, the following terms have the following defined meanings:
The term "Dalton" or "Da" as used herein has the meaning, the unit used for indicating mass on an atomic or molecular scale. One Dalton is one twelfth of the mass of an unbound neutral atom of carbon-12 in its nuclear and electronic ground state, having a value of 1.660538921(73)×10-27 kg.

The term "Angstrom" as used herein has the meaning, the unit of length equal to 10⁻¹⁰ meters (i.e., 0.1 nm).

The term "dialysate" as used herein has the meaning, a fluid used in dialysis to remove waste material from the blood of the patient, including fluids that replenish certain elements of blood and/or prevent certain elements from leaving the blood (e.g., electrolytes and/or water). Dialysate may include a solution of sodium, calcium, chloride, potassium, and magnesium. Dialysate may also include glucose and lactate, just to name a few non-limiting examples.

The term "filtrate" as used herein has the meaning, the material (e.g., a liquid) that has passed through a filter.

The term "fluid flow path" as used herein has the meaning, the route along which a fluid moves when the device is in use.

The term "forward osmosis" as used herein has the meaning, diffusion of a solvent through a semi-permeable membrane from a solution of low osmotic pressure (i.e., high solvent concentration) to a solution with a higher osmotic pressure (i.e., low solvent concentration). Forward osmosis includes, but is not limited, processes which equalize the osmotic pressure on both sides of the semi-permeable membrane.

The term "nominal molecular weight limit" (NMWL), sometimes referred to as molecular weight cutoff (MWCO), as used herein has the meaning, the lowest molecular weight of a dilute globular solute (measured in Daltons) in which 90% of the solute is retained by the membrane as determined using the method taught by K.J. Kim et al, A comparative study of techniques used for porous membrane characterization: pore characterization, 87 Journal of Membrane Science 35-46 (1994) using dextran and a transmembrane pressure of 50 kPa.

The term "osmotic membrane" as used herein has the meaning, a semipermeable membrane that is impermeable or less permeable to large molecules, such as proteins and polysaccharides, and certain polar molecules such as urea and creatinine while being permeable to small molecules such as water in the direction of an osmotic gradient.

The term "permeate fluid" as used herein has the meaning, the fluid that passes through the pores or interstices of a filter or membrane.

The term "regenerate" as used herein has the meaning, to restore towards its original/new useful form. In the context of this disclosure, regenerating dialysate includes but is not limited to removing at least some waste products transferred into a dialysate solution from the patient's during dialysis, such as urea and/or creatinine. Regenerating dialysate may also include adding components to the dialysate to replenish elements transferred from the dialysate into the patient, such as electrolytes.

The term "retentate" as used herein has the meaning, the material (e.g., a solid) that does not pass through the pores or interstices of a filter or membrane.

The term "reverse osmosis" as used herein has the meaning, diffusion of a solvent through a semi-permeable membrane from a solution of high osmotic pressure (i.e., low solvent concentration) to a solution with a lower osmotic pressure (i.e., high solvent concentration).

Further forms, objects, features, aspects, benefits, advantages, and embodiments of the present invention will become apparent from a detailed description and drawings provided herewith.

### BRIEF DESCRIPTION OF THE DRAWINGS

FIG. 1 is a left view of an exemplary dialysate regenerating device.
FIG. 2 is a cross-sectional, right view of the device of FIG. 1.
FIG. 3 is a perspective view of another exemplary dialysate regenerating assembly.
FIG. 4 is an exploded view of the first device of the assembly of FIG. 3.
FIG. 5 is bottom, perspective view of a portion of the first device of FIG. 3.
FIG. 6 is an exploded view of the second device of the assembly of FIG. 3.
FIG. 7 is a side view of another embodiment of a dialysate regenerating device.
FIG. 8 is an exploded view of the dialysate regenerating device of FIG. 7.
FIGs. 9 and 10 are perspective views of the first portion of the dialysate regenerating device of FIG. 7 and 8.
FIG. 11 is a side view of the first portion of the dialysate regenerating device of FIGs. 7 and 8.
FIGs. 12 and 13 are perspective views of the second potion of the dialysate regenerating device of FIGs. 7 and 8.
FIG. 14 is a side view of the second portion of the dialysate regenerating device of FIGs. 7 and 8.
FIG. 15 is a bottom view of the second portion of the dialysate regenerating device of FIGs. 7 and 8.
FIG. 16 is a cross-sectional view along line 16-16 of FIG. 15.
FIG. 17 is a cross-sectional view along line 17-17 of FIG. 15.
FIGs. 18 and19 are perspective views of the third portion of the dialysate regenerating device of FIGs. 7 and 8.
FIG. 20 is a side view of the third portion of the dialysate regenerating device of FIGs. 7 and 8.

### DESCRIPTION OF THE SELECTED EMBODIMENTS

For the purpose of promoting an understanding of the principles of the invention, reference will now be made to the embodiments illustrated in the drawings and specific language will be used to describe the same. It will nevertheless be understood that no limitation of the scope of the invention is thereby intended. Any alterations and further modifications in the described embodiments, and any further applications of the principles of the invention as described herein are contemplated as would normally occur to one skilled in the art to which the invention relates. One embodiment of the invention is shown in great detail, although it will be apparent to those skilled in the relevant art that some features that are not relevant to the present invention may not be shown for the sake of clarity.

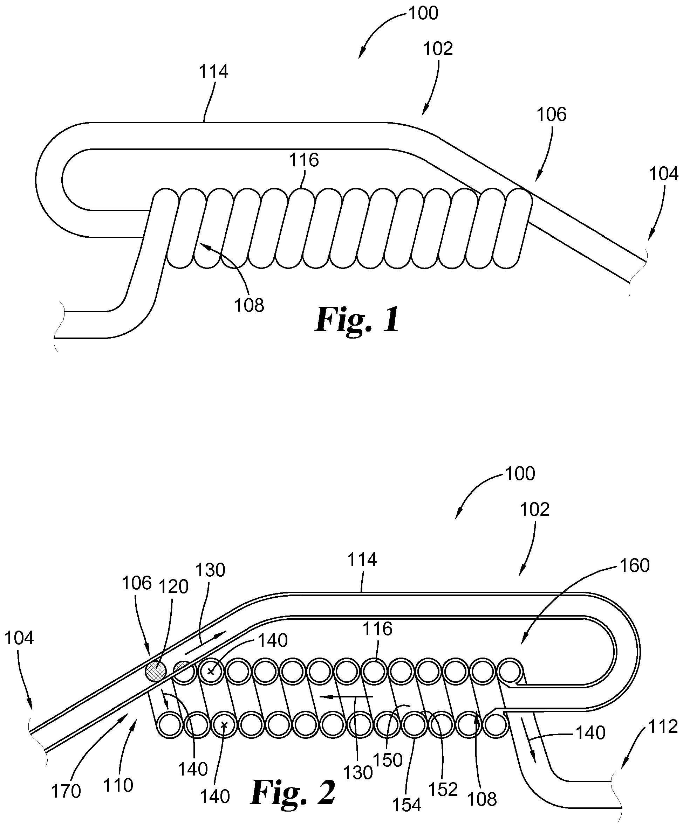

Figures 1 and 2 illustrate an exemplary dialysate regenerating device. Dialysate regenerating device 100 includes a device body 102 defining a fluid inlet 104, a first stage 106, a second stage 108, a first outlet 110, and a second outlet 112. Dialysate regenerating device 100 may include one or more tubes in its construction. In some instances, dialysate regenerating device 100 includes a first tube 114 and a second tube 116 joined to one another, such as by thermal bonding and/or chemical bonding just to name a few non-limiting examples.

First stage 106 of dialysate regenerating device 100 includes a filter membrane 120 separating a first fluid flow path 130 from a second fluid flow path 140. In the illustrated embodiment, filter membrane 120 is arranged in a crossflow (i.e., tangential flow) configuration. For example, filter membrane 120 extends across an opening in the wall of first tube 114, such as the opening to second fluid flow path 140. A portion of dialysate fluid flowing from fluid inlet 104 passes across a face of filter membrane 120 and another portion passes through filter membrane 120. The portion of dialysate fluid that passes across a face of filter membrane 120 enters first fluid flow path 130 while the portion flowing through filter membrane 120 enters second fluid flow path 140. As will be appreciated by those of ordinary skill in the art, filter membrane 120 has a thickness and the portion of fluid that flows through the filter membrane passes through the thickness of the filter membrane through pores or openings of the filter membrane whereas the portion of dialysate fluid that passes across the face of the filter membrane does not pass through the thickness of the filter membrane. Advantageously, this type of filtration can reduce the accumulation of material (i.e., foulant) on the surface of the filter membrane.

Second stage 108 of dialysate regenerating device 100 includes an osmotic membrane 150 separating first fluid flow path 130 from the second fluid flow path 140. In the illustrated embodiment, osmotic membrane 150 is included in the wall of second tube 116 and allows water to pass through from second fluid flow path 140 into first fluid flow path 130. Osmotic membrane 150 may be positioned between the first fluid flow path 130 and second fluid flow path 140, between second fluid flow path 140 and the environment adjacent to the device body, or both. For example, inner wall 152 of second tube 116 may include osmotic membrane 150, outer wall 154 of second tube 116 may include osmotic membrane 150, or both inner wall 152 and outer wall 154 may include osmotic membrane 150.

In some arrangements, electrodes or ionically charged species may be incorporated in second stage 108 to sequester dissolved ions and/or for driving osmosis in the desired direction. For example, electrodes or ionically charged species may be incorporated in the osmotic membrane 150.

In some instances, dialysate regenerating device 100 is arranged to prevent water transport between the device and environment adjacent to the device body (e.g., the peritoneal cavity). For example, dialysate regenerating device 100 may be encapsulated within an impermeable membrane. Alternatively, portions of dialysate regenerating device 100 may include and/or be covered with a permeable material.

In the illustrated embodiment, second tube 116 extends helically around first fluid flow path 130 in second stage 108. Additionally, first fluid flow path 130 and second fluid flow path 140 are arranged in a counter-flow arrangement in second stage 108. As shown in figure 2, first fluid flow path 130 enters second stage 108 at first end 160 and exits second stage 108 at second end 170. Second fluid flow path 140, however, enters second stage 108 at second end 170 and exists second stage 108 at first end 160. Accordingly, first fluid flow path 130 and second fluid flow path 140 extend in opposing directions. Advantageously, such an arrangement can provide for a greater osmotic gradient between fluid of first fluid flow path 130 and fluid of the second fluid flow path 140 along a length of the second stage than configurations having parallel fluid flow.

Variations on the above illustrated embodiment have been contemplated. For instance, while in the above embodiment the osmotic membrane is included in the wall of the second tube, the osmotic membrane may be included in the wall of the first tube. Alternatively, or additionally, the osmotic membrane may be a separate component (e.g., a tube) secured to the first tube or second tube. Similarly, while the second tube 116 is shown as extending helically around first fluid flow path 130, the first tube may extend helically around second fluid flow path 140 or the first and second fluid flow paths may form a double-helix, including while extending in a counter-flow arrangement.

In some instances, helically wound tube 116 provides a fluid-tight seal sufficient to retain the fluid within first fluid flow path 130 from escaping between adjacent coils. Adjacent coils can be fused together, such as by thermal or chemical bonding. Additionally, the portion of first tube 114 meeting first end 160 of second stage 108 may form a fluid-tight seal to retain fluid within first fluid flow path 130 from escaping between first tube 114 and second tube 116. In some instances, first tube 114 and second tube 116 are thermally or chemically bonded to one another. It should also be appreciated that a third tube (not illustrated) can be coupled to second end 170 of second stage 108 to retain fluid of first fluid flow path 130 exiting second stage 108.

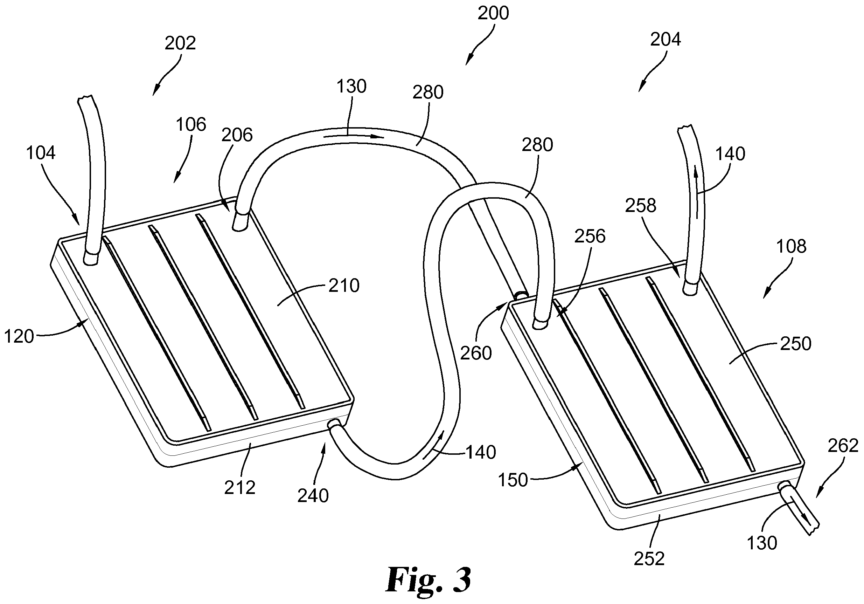

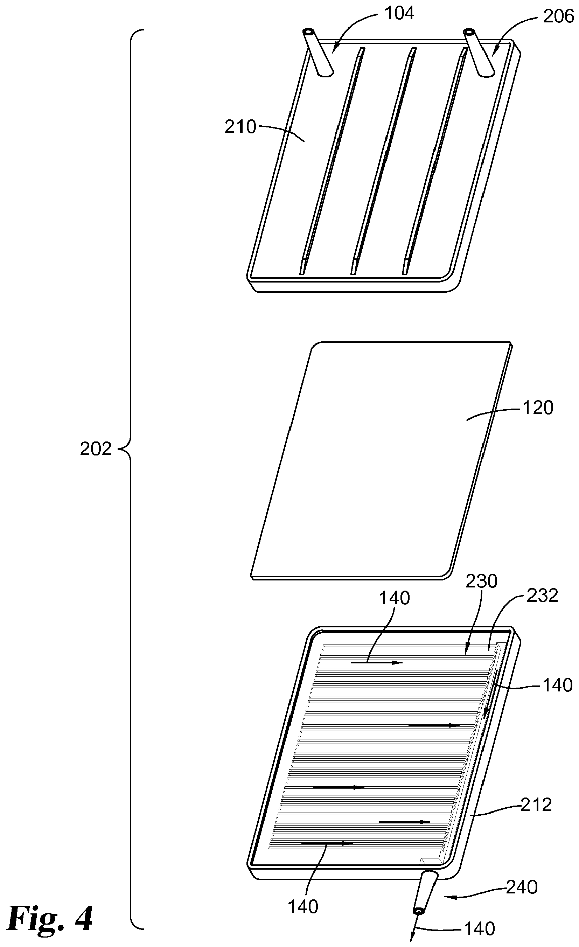

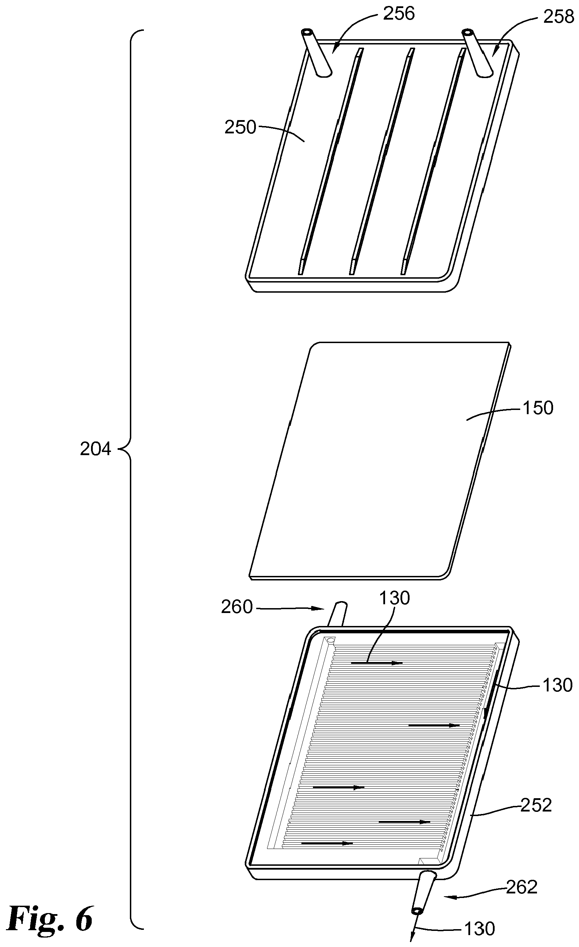

Figures 3-6 illustrate another exemplary dialysate regenerating assembly 200. Dialysate regenerating device 200 includes a first device 202 and a second device 204. First device 202 houses first stage 106 of dialysate regenerating device 200 and includes a first portion 210 and a second portion 212 separated, at least in part, by filter membrane 120.

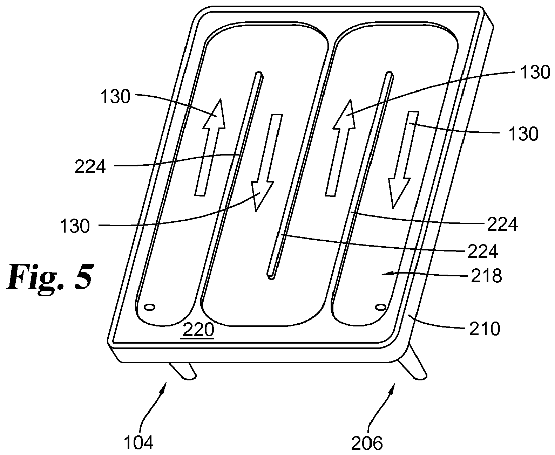

In first device202, first portion 210 defines fluid inlet 104 and first portion outlet 206. Also in first device202, first portion 210 and filter membrane 120 define a first fluid flow path 130. In some instances, first fluid flow path 130 is defined, at least in part, by a groove or channel 218 in an inward-facing surface 220 of first portion 210. In certain arrangements, first portion 210 includes flow channel walls 224 that separate portions of first fluid flow path 130. For example, flow channel walls 224 may separate portions of first fluid flow path 130. In the illustrated embodiment, flow channel walls 224 separate portions of first fluid flow path 130 that extend in opposing directions. As shown in figure 5, first fluid flow path 130 extends along a serpentine path, with flow channel walls 224 separating portions of first flow path 130. In this way, a fluid flowing between fluid inlet 104 and first portion outlet 206 along first fluid flow path 130 traverses a greater distance along filter membrane 120 than if the fluid were allowed to flow along a straight path extending between fluid inlet 104 and first portion outlet 206.

As discussed above, first stage 106 of dialysate regenerating device 100 includes a filter membrane 120 separating a first fluid flow path 130 from a second fluid flow path 140. In dialysate regenerating device 200, filter membrane 120 is arranged in a crossflow (i.e., tangential flow) configuration. A portion of dialysate fluid flowing from fluid inlet 104 passes across a face of filter membrane 120 and another portion passes through filter membrane 120. The portion of dialysate fluid that passes across a face of filter membrane 120 enters first fluid flow path 130 while the portion flowing through filter membrane 120 enters second fluid flow path 140. As will be appreciated by those of ordinary skill in the art, filter membrane 120 has a thickness and the portion of fluid that flows through the filter membrane passes through the thickness of the filter membrane through pores or openings of the filter membrane whereas the portion of dialysate fluid that passes across the face of the filter membrane does not pass through the thickness of the filter membrane. Advantageously, this type of filtration can reduce the accumulation of material (i.e., foulant) on the surface of the filter membrane.

Second portion 212 also has an inward-facing surface defining channels 230 separated by channel walls 232. Channels 230 define second fluid flow path 140 which leads to second portion outlet 240. Channels 230 and channel walls 232 are arranged to direct permeate fluid (i.e., fluid that passes through the thickness of filter membrane 120) to second portion outlet 240.

Second device204 is similar to first device202 and includes a first portion 250, a second portion 252, and an osmotic membrane 150 positioned between the first portion 250 and second portion 252 and separating the first fluid flow path 130 and second fluid flow path 140. First portion 250 includes a first portion inlet 256 and a first portion outlet 258. Second portion 252 includes a second portion inlet 260 and second portion outlet 262.

First portion 250 and second portion 252 can define grooves or channels such as those described with respect to first portion 210 and second portion 212 of first device202. Accordingly, fluid entering an inlet of the first or second portion travels along osmotic membrane 150 as it travels towards an outlet of the same portion. In the illustrated embodiment, first fluid flow path 130 enters second portion inlet 260 and travels towards second portion outlet 262 while second fluid flow path 140 enters first portion inlet 256 and travels towards first portion outlet 258.

Osmotic membrane 150 allows the transfer of water between first fluid flow path 130 and second fluid flow path 140, in particular, through forward osmosis. In the illustrated embodiment, water can transfer from second fluid flow path 140 in first portion 250 through osmotic membrane 150 into first fluid flow path 130 of second portion 252. In this way, water can be added to dialysate fluid in first fluid flow path 130 before returning to a dialysis machine or to the patient (e.g., the peritoneum of the patient).

The illustrated embodiment shows tubing 280 connecting second portion outlet 240 to first portion inlet 256 and first portion outlet 206 to second portion inlet 260. Accordingly, second portion outlet 252 is in fluid communication with first fluid flow path 130 and can also be in fluid communication with the peritoneum or a dialysis machine and/or cycler to return regenerated dialysate fluid. First portion outlet 258 is illustrated in fluid communication with second fluid flow path 140 and, therefore, maybe in fluid communication with a lumen or container for waste material (e.g., an extracorporeal drainage bag) or, in some instances, to the urinary tract of the patient (e.g., a ureter). However, one of ordinary skill will recognize that outlets of first device202 can be connected to inlets of second device204 in a number of configurations, with the outlets of the second device204 being arranged accordingly.

Methods of regenerating a dialysate and/or using a dialysate regenerating device will now be described. Reference may be made to figures 1 and 2 for ease of understanding; however, the methods are not limited to the device(s) of those figures. Methods includes filtering a dialysate fluid into a permeate fluid and retentate fluid with a filter membrane having a nominal molecular weight limit (NMWL) or molecular weight cutoff (MWCO) of 500 kDa or less, and passing water from the permeate fluid back into the retentate fluid through an osmotic membrane separate from the filter membrane and having a smaller average pore size or no discernable pores. The permeate fluid being fluid that passes through the filter membrane and the retentate fluid being fluid that does not pass through the filter membrane. Importantly, passing water from the permeate fluid into the retentate fluid through the osmotic membrane can include passing water via forward-osmosis (e.g., assisted by forward-osmosis or via forwards osmosis only). In some instances, passing water via forward-osmosis is assisted by a pressure gradient.

In one exemplary method of using a dialysate regenerating device, the method includes passing a dialysate fluid through a fluid opening and a first stage of the dialysate regenerating device, the first stage including a filter membrane having a nominal molecular weight limit (NMWL) or molecular weight cutoff (MWCO) of 500 kDa or less. The method also includes passing the dialysate fluid through a second stage of the dialysate regenerating device, the second stage including an osmotic membrane separate from the filter membrane and having an average pore size that is smaller than that of the filter membrane. The second stage may also include passing water through the osmotic membrane via forward-osmosis (e.g., assisted by forward-osmosis or via forward-osmosis only). In some instances, passing water via forward-osmosis is assisted by a pressure gradient.

In the embodiments illustrated in figures 1 and 2, dialysate fluid enters fluid inlet 104 and flows towards first stage 106. As dialysate fluid passes over filter membrane 120, a portion of the dialysate, the retentate, continues into first fluid flow path 130 and first tube 114. A portion of the dialysate, the permeate, flows through the thickness of filter membrane 120 and into second fluid flow path 140 and second tube 116. The retentate continues along first fluid flow path 130 into first end 160 of second stage, and the permeate enters second end 170 of second stage 108. In second stage 108, first fluid flow path 130 extends through a central opening defined by helically wound second fluid flow path 140 and towards first outlet 110. As the permeate flows through second stage 108 along first fluid flow path 130 and the retentate flows through second stage 108 along second fluid flow path 140, water moves from the permeate fluid in second tube 116 through the osmotic membrane 150 and into the retentate fluid through forward osmosis. Retentate fluid with water that has passed from the permeate through the osmotic membrane then exits the dialysate regenerating device at the first outlet 110. Permeate fluid that did not pass through the osmotic membrane remains in second tube 116 and exits the device at second outlet 112.

In the illustrated embodiment, fluid inlet 104 and first fluid outlet 110 are preferably in fluid communication with a pump supplying fluid, such as from the peritoneum. Second fluid outlet 112 can lead to a drainage system (e.g., an extracorporeal drainage bag) or, in some instances, to the urinary tract of the patient (e.g., a ureter). However, as will be appreciated by one skilled in the art, this arrangement may vary based on the filter membrane configuration in the first stage.

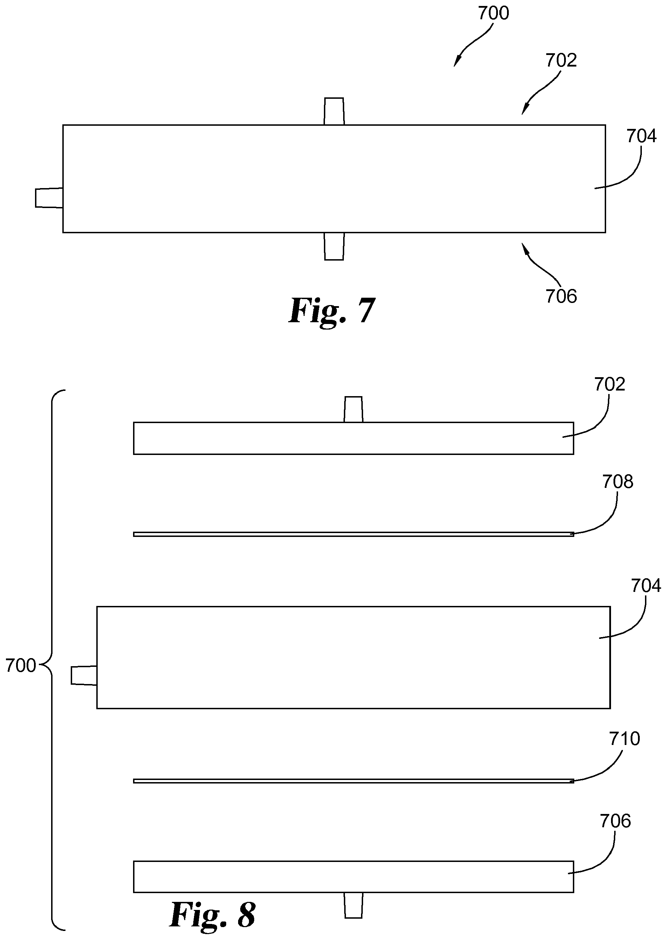

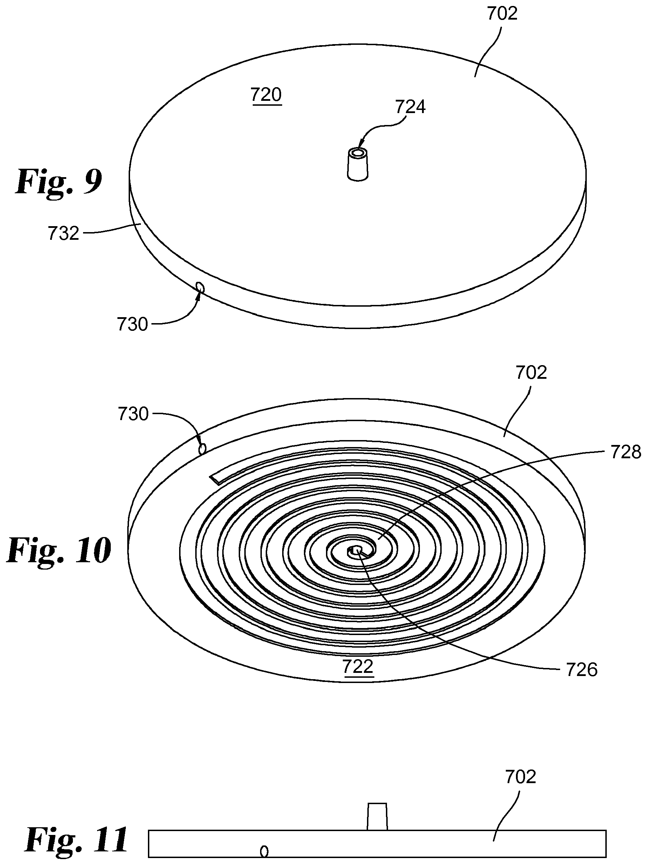

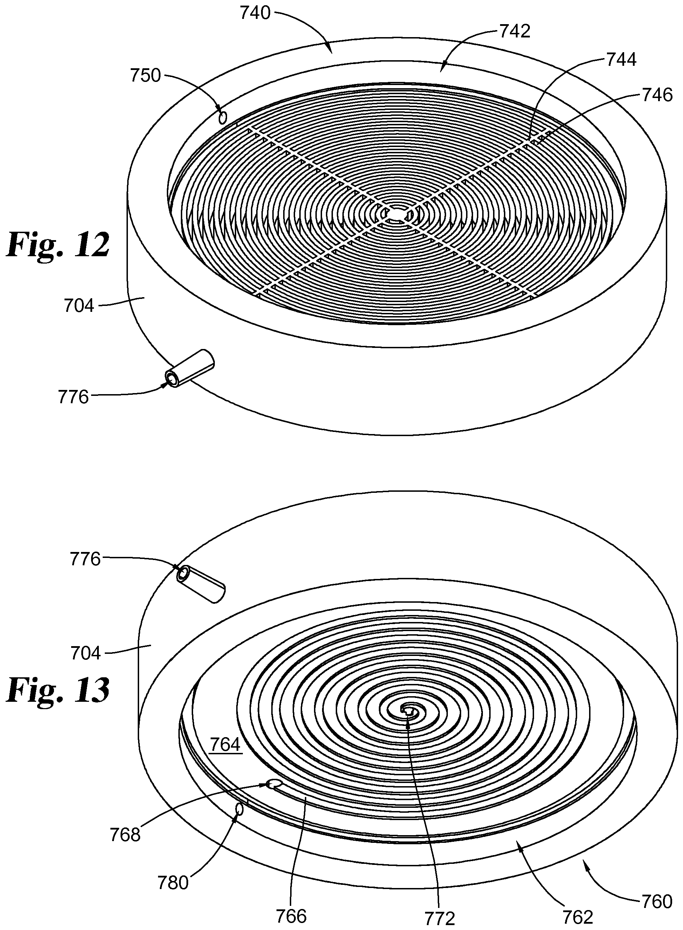

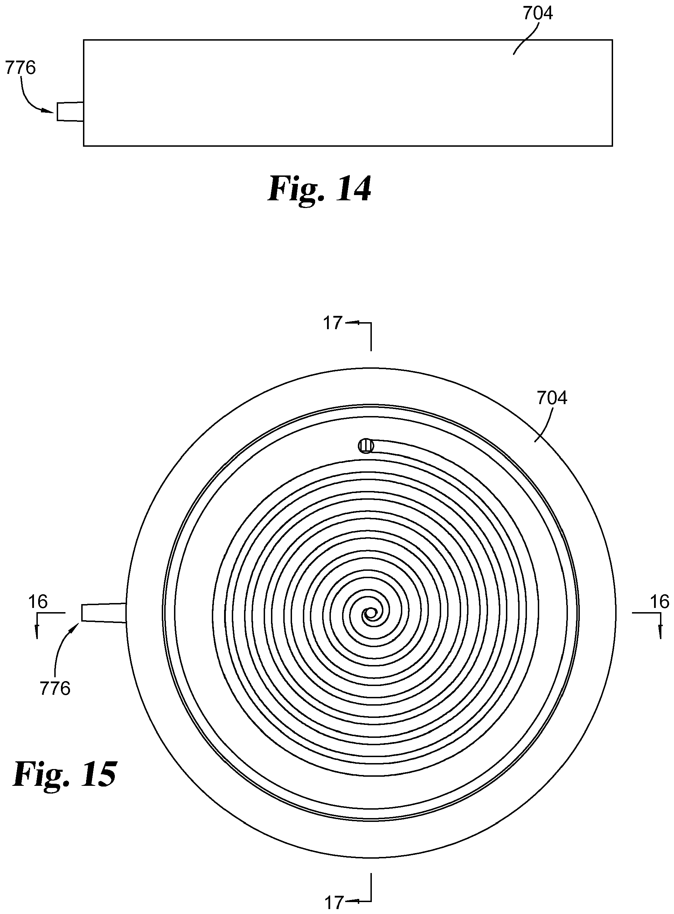

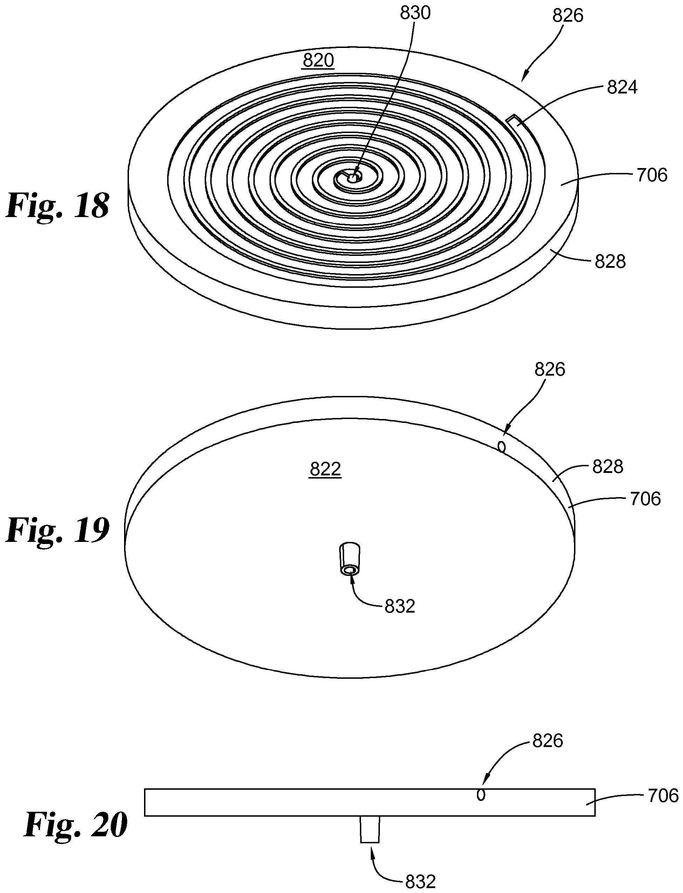

Figures 7-21 illustrate another embodiment of a dialysate regenerating device 700. Dialysate regenerating device 700 includes a first portion 702, a second portion 704, and a third portion 706. Positioned between first portion 702 and second portion 704 is a first membrane 708 for the first stage of dialysate regenerating device 700, and positioned between second portion 704 and third portion 706 is a second membrane 710 for the second stage of dialysate regenerating device 700.

First portion 702 has an upper surface 720 and a lower surface 722. Upper surface 720 defines an opening 724 to a fluid passageway 726. Fluid passageway 726 extends through the thickness of the first portion 702. Lower surface 722 defines a channel 728. Channel 728 is in fluid communication with fluid passageway 726 and a fluid outlet 730 in a side surface 732 of the first portion 702. In some instances, channel 728 extends along a helical path along lower surface 722.

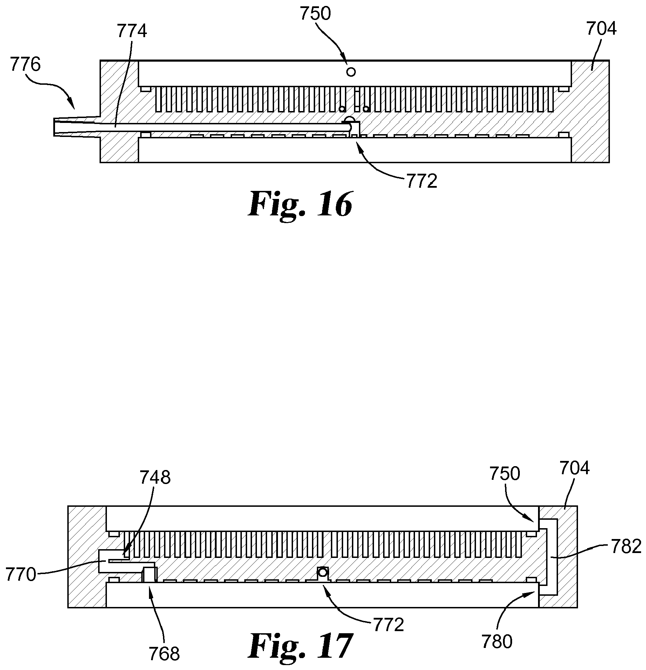

Second portion 704 has a first side 740 that defines a recess 742 arranged to receive first portion 702 and first membrane 708. An upward facing surface 744 of second portion 704 defines a channel 746 or plurality of channels in fluid communication with an outlet 748 (shown in Figure 17). First side 740 of second portion 704 also defines an inlet 750 arranged to align with fluid outlet 730 of first portion 702 when first portion 702 is received within recess 742.

A second side 760 of second portion 704 defines a recess 762 arranged to receive second membrane 710 and third portion 706. A downward facing surface 764 of second portion 704 defines a channel 766 with an outlet 768 in fluid communication with outlet 748 of first side 740 through passageway 770. Second side 760 of second portion 704 also defines an outlet 772 in fluid communication with channel 766. Outlet 772 communicates with passageway 774 and outlet 776 of second portion 704. Second side 760 of second portion 704 also defines a fluid outlet 780 which is in fluid communication with a passageway 782 and fluid inlet 750 of first side 740.

Third portion 706 has an upper surface 820 and a lower surface 822. Upper surface 820 defines a channel 824 extending between and in fluid communication with a fluid inlet 826 in a side surface 828 of the third portion 706 and a fluid passageway 830. Fluid passageway 830 extends through the thickness of third portion 706 and communicates with fluid outlet 832 on the lower surface 822 of the third portion 706.

Similar to channel 728 of first portion 702, channel 824 of third portion 706 may extend along a helical path. In some instances, channel 824 aligns with channel 766 of second portion 704, separated by second membrane 710 when assembled. For example, channel 766 of second portion 704 and channel 824 of third portion 706 may be helically aligned when the third portion 706 is received within recess 762 of second portion 704.

During operation of the device of Figures 7-21, a feed solution (e.g., a used dialysate) is provided to opening 724 of first portion 702. The feed solution travels from upper surface 720 to lower surface 722 of first portion 702 through fluid passageway 726. On lower surface 722, the feed solution passes through channel 728 and along the upper surface of first membrane 708. As the feed solution passes along first membrane 708, permeate of the first stage passes through the first membrane 708, channel 746, outlet 748 and enters passageway 770, and retentate of the first stage passes through channel 728, fluid outlet 730, fluid inlet 750 and passageway 782.

After passing through passageway 770, the permeate of the first stage exits outlet 768 and becomes the feed solution for the second stage. The feed solution for the second stage then flows through channel 766 along the top surface of second membrane 710. Similarly, after passing through passageway 782, the retentate of the first stage flows through outlet 780 and becomes the draw solution for the second stage. The draw solution then flows through inlet 826 and channel 824 along the bottom surface of second membrane 710.

As the feed solution flows along the top surface of second membrane 710 and the draw solution flows along the bottom surface of second membrane 710, solvent from one side of the membrane diffuses to the other side. For example, water from feed solution may transfer through the second membrane 710 into the draw solution. The draw solution for the second stage exits outlet 832 with permeate that has transferred through second membrane 710 from the feed solution. Similarly, the retentate for the second stage flows through passageway 774 and exits outlet 776. In some embodiments, the solution exiting outlet 832 is regenerated dialysate while the solution exiting outlet 776 is waste solution.

As will be appreciated by one of ordinary skill in the art, the draw and feed solutions of the second stage in the embodiment illustrated in Figures 7-21 can be reversed, with the draw solution (i.e., the retentate of the first stage) communicating with the top surface of second membrane 710 and the feed solution (i.e., the permeate of first stage) communicating with the bottom surface of second membrane 710. The solutions flowing out of outlet 832 and outlet 776 would simply be reversed in such a configuration, with the regenerated dialysate exiting outlet 776 and the waste solution exiting outlet 832.

### Listing of Certain Embodiments:

1. A method, comprising:
   filtering a dialysate fluid into a permeate fluid and retentate fluid with a filter membrane having a nominal molecular weight limit (NMWL) of 500 kDa or less; and
   passing water from the permeate fluid into the retentate fluid through an osmotic membrane separate from the filter membrane and having a lower nominal molecular weight limit (NMWL) than the nominal molecular weight limit (NMWL) of the filter membrane.
2. The method of embodiment 1, wherein:
   passing water from the permeate fluid into the retentate fluid through an osmotic membrane includes passing water from the permeate fluid into the retentate fluid using forward osmosis or partial forward osmosis.
3. The method of embodiment 1 or 2, wherein:
   the filter membrane has a nominal molecular weight limit (NMWL) of 100 kDa or less.
4. The method of embodiment 1 or 2, wherein:
   the filter membrane has a nominal molecular weight limit (NMWL) of 10 kDa or less.
5. The method of any preceding embodiment, wherein:
   the nominal molecular weight limit (NMWL) of the osmotic membrane is less than 80% of the nominal molecular weight limit (NMWL) of the filter membrane.
6. The method of any one of embodiments 1 to 4, wherein:
   the nominal molecular weight limit (NMWL) of the osmotic membrane is less than 50% of the nominal molecular weight limit (NMWL) of the filter membrane.
7. The method of any one of embodiments 1 to 4, wherein:
   the nominal molecular weight limit (NMWL) of the osmotic membrane is less than 30% of the nominal molecular weight limit (NMWL) of the filter membrane.
8. The method of any one of embodiments 1 to 4, wherein:
   the nominal molecular weight limit (NMWL) of the osmotic membrane is less than 10% of the nominal molecular weight limit (NMWL) of the filter membrane.
9. The method of any preceding embodiment, wherein:
   the osmotic membrane is a hydrophilic membrane.
10. A device for regenerating a dialysate fluid, comprising:
   a device body defining a dialysate fluid inlet, a first stage, a second stage, a first outlet, and a second outlet;
   the first stage having a filter membrane separating a first fluid flow path from a second fluid flow path, the first fluid flow path extending from the first stage to the first outlet and the second fluid flow path extending from the first stage to the second outlet;
   the second stage having an osmotic membrane separating the first fluid flow path from the second fluid flow path; and
   wherein dialysate fluid enters the dialysate fluid inlet, passes through the first stage which separates the dialysate fluid into the first fluid flow path and the second fluid flow path, and passes through the second stage which allows solvent to pass through the osmotic membrane from the second fluid flow path into the first fluid flow path.
11. The device of embodiment 10, wherein:
   in the second stage, the first fluid flow path extends in a direction opposing that of the second fluid flow path.
12. The device of embodiment 10 or 11, wherein:
   in the second stage, the second fluid flow path extends helically around the first fluid flow path.
13. The device of embodiment 10 or 11, wherein:
   in the second stage, the first fluid flow path extends helically around the second fluid flow path.
14. The device of embodiment 10 or 11, wherein
   in the second stage, the first and second fluid flow paths form a double helix.
15. The device of any one of embodiments 10 to 14, wherein:
   a portion of the dialysate fluid passing through the filter membrane enters the second fluid flow path and a portion of dialysate fluid that does not pass through the filter membrane continues into the first fluid flow path.
16. The device of any one of embodiments 10 to 15, wherein:
   the filter membrane has a nominal molecular weight limit (NMWL) of 500 kDa or less.
17. The device of any one of embodiments 10 to 15, wherein:
   the filter membrane has a nominal molecular weight limit (NMWL) of 100 kDa or less.
18. The device of any one of embodiments 10 to 15, wherein:
   the filter membrane has a nominal molecular weight limit (NMWL) of 10 kDa or less.
19. The device of any one of embodiments 10 to 18, wherein:
   the osmotic membrane and the filter membrane each have a nominal molecular weight limit (NMWL); and
   wherein the nominal molecular weight limit (NMWL) of the osmotic membrane is less than 80% of the nominal molecular weight limit (NMWL) of the filter membrane.
20. The device of any one of embodiments 10 to 18, wherein:
   the osmotic membrane and the filter membrane each have a nominal molecular weight limit (NMWL); and
   wherein the nominal molecular weight limit (NMWL) of the osmotic membrane is less than 50% of the nominal molecular weight limit (NMWL) of the filter membrane.
21. The device of any one of embodiments 10 to 18, wherein:
   the osmotic membrane and the filter membrane each have a nominal molecular weight limit (NMWL); and
   wherein the nominal molecular weight limit (NMWL) of the osmotic membrane is less than 30% of the nominal molecular weight limit (NMWL) of the filter membrane.
22. The device of any one of embodiments 10 to 18, wherein:
   the osmotic membrane and the filter membrane each have a nominal molecular weight limit (NMWL); and
   wherein the nominal molecular weight limit (NMWL) of the osmotic membrane is less than 10% of the nominal molecular weight limit (NMWL) of the filter membrane.
23. The device of any one of embodiments 10 to 22, wherein:
   the osmotic membrane is a hydrophilic membrane.
24. The device of any one of embodiments 10 to 23, wherein:
   the device body has an outer surface allowing water transport from the first fluid flow path or second fluid flow path to an environment adjacent the device body or vice versa.

While the invention has been illustrated and described in detail in the drawings and foregoing description, the same is to be considered as illustrative and not restrictive in character, it being understood that only the preferred embodiment has been shown and described and that all changes, equivalents, and modifications that come within the spirit of the inventions defined by following claims are desired to be protected. All publications, patents, and patent applications cited in this specification are herein incorporated by reference as if each individual publication, patent, or patent application were specifically and individually indicated to be incorporated by reference and set forth in its entirety herein.

## Claims

1. A method, comprising:
filtering a dialysate fluid into a permeate fluid and retentate fluid with a filter membrane having a nominal molecular weight limit (NMWL) of 500 kDa or less; and
passing water from the permeate fluid into the retentate fluid through an osmotic membrane separate from the filter membrane and having a lower nominal molecular weight limit (NMWL) than the nominal molecular weight limit (NMWL) of the filter membrane.

2. The method of claim 1, wherein:
passing water from the permeate fluid into the retentate fluid through an osmotic membrane includes passing water from the permeate fluid into the retentate fluid using forward osmosis or partial forward osmosis.

3. The method of claim 1 or 2, wherein:
the filter membrane has a nominal molecular weight limit (NMWL) of 100 kDa or less.

4. The method of claim 1 or 2, wherein:
the filter membrane has a nominal molecular weight limit (NMWL) of 10 kDa or less.

5. The method of any preceding claim, wherein:
the nominal molecular weight limit (NMWL) of the osmotic membrane is less than 80% of the nominal molecular weight limit (NMWL) of the filter membrane.

6. The method of any one of claims 1 to 4, wherein:
the nominal molecular weight limit (NMWL) of the osmotic membrane is less than 50% of the nominal molecular weight limit (NMWL) of the filter membrane.

7. The method of any one of claims 1 to 4, wherein:
the nominal molecular weight limit (NMWL) of the osmotic membrane is less than 30% of the nominal molecular weight limit (NMWL) of the filter membrane.

8. The method of any one of claims 1 to 4, wherein:
the nominal molecular weight limit (NMWL) of the osmotic membrane is less than 10% of the nominal molecular weight limit (NMWL) of the filter membrane.

9. The method of any preceding claim, wherein:
the osmotic membrane is a hydrophilic membrane.

10. A device for regenerating a dialysate fluid, comprising:
a device body defining a dialysate fluid inlet, a first stage, a second stage, a first outlet, and a second outlet;
the first stage having a filter membrane separating a first fluid flow path from a second fluid flow path, the first fluid flow path extending from the first stage to the first outlet and the second fluid flow path extending from the first stage to the second outlet;
the second stage having an osmotic membrane separating the first fluid flow path from the second fluid flow path; and
wherein dialysate fluid enters the dialysate fluid inlet, passes through the first stage which separates the dialysate fluid into the first fluid flow path and the second fluid flow path, and passes through the second stage which allows solvent to pass through the osmotic membrane from the second fluid flow path into the first fluid flow path.

11. The device of claim 10, wherein:
in the second stage, the first fluid flow path extends in a direction opposing that of the second fluid flow path.

12. The device of claim 10 or 11, wherein:
in the second stage, the second fluid flow path extends helically around the first fluid flow path.

13. The device of claim 10 or 11, wherein:
in the second stage, the first fluid flow path extends helically around the second fluid flow path.

14. The device of claim 10 or 11, wherein
in the second stage, the first and second fluid flow paths form a double helix.

15. The device of any one of claims 10 to 14, wherein:
a portion of the dialysate fluid passing through the filter membrane enters the second fluid flow path and a portion of dialysate fluid that does not pass through the filter membrane continues into the first fluid flow path.
